(19) **Europäisches Patentamt / European Patent Office / Office européen des brevets**

(11) **EP 4 692 194 A1**

(12) **EUROPEAN PATENT APPLICATION**
published in accordance with Art. 153(4) EPC

(43) Date of publication:
**11.02.2026 Bulletin 2026/07**

(21) Application number: 24797212.8

(22) Date of filing: **26.04.2024**

(51) International Patent Classification (IPC):
**C08J 11/16** (2006.01)    **C07C 27/02** (2006.01)
**C07C 31/20** (2006.01)    **C07C 63/26** (2006.01)
**C08J 11/24** (2006.01)

(52) Cooperative Patent Classification (CPC):
Y02W 30/62

(86) International application number:
**PCT/JP2024/016555**

(87) International publication number:
**WO 2024/225470 (31.10.2024 Gazette 2024/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.04.2023 JP 2023075315**

(71) Applicant: **KIRIN HOLDINGS KABUSHIKI KAISHA
Nakano-ku, Tokyo 164-0001 (JP)**

(72) Inventors:
• **TANIGUCHI, Yoshimasa**
  **Tokyo 164-0001 (JP)**
• **MIWA. Mayuka**
  **Tokyo 164-0001 (JP)**
• **MATSUKURA,Yasuko**
  **Tokyo 164-0001 (JP)**

(74) Representative: **Hoffmann Eitle
Patent- und Rechtsanwälte PartmbB
Arabellastraße 30
81925 München (DE)**

(54) **METHOD FOR DEPOLYMERIZING POLYESTER**

(57)    A method for depolymerizing a polyester, the method comprising:
forming a mixture comprising the following components (a), (b), and (c):
(a) a polyester;
(b) one or more alkali compounds selected from the group consisting of an alkali metal hydroxide, an alkali metal carbonate, an alkali metal bicarbonate, an alkaline earth metal hydroxide, an alkaline earth metal carbonate, and an alkaline earth metal bicarbonate; and
(c) a solvent comprising at least one of an alcohol-based solvent or a ketone-based solvent,

and depolymerizing the polyester in the mixture. A temperature of the mixture at a time when the mixture comprising an entire amount of the polyester, the alkali compound, and the solvent is formed is 75°C or less. A ratio of an amount of the polyester to an amount of the solvent is from 0.2 to 20 on a mass basis.

**Description**

**Technical Field**

The present invention relates to a method for depolymerizing a polyester.

**Background Art**

Recently, environmental pollution caused by plastic waste has become a problem. In order to reduce the environmental load, there is an urgent need to develop fundamental technologies for efficiently recycling plastic products. Among plastic products, polyester is used in large quantities for beverage bottles or fibers. Therefore, there is a strong demand for the establishment of an efficient recycling method.

As recycling methods for used polyester products, material recycling and chemical recycling are known. Material recycling is a method of sorting used polyester products and then melting and re-molding them as resin, but it is difficult to restore the quality to that before recycling because impurities cannot be completely removed. On the other hand, chemical recycling, by chemically decomposing the polyester into low-molecular-weight compounds, makes it possible in principle to produce products of a quality equivalent to that of the original polyester products.

Chemical recycling methods for polyester are broadly classified into a hydrolysis method using water as a solvent, an alcoholysis method using an alcohol as a solvent, and a glycolysis method using a glycol as a solvent. In addition to these three types, in recent years, the use of microorganisms to perform depolymerization has also been considered.

The hydrolysis method is a method of decomposing polyethylene terephthalate into terephthalic acid and ethylene glycol by reacting a polyethylene terephthalate melt with steam and then with ammonium hydroxide. This method has the advantage of not requiring the use of glycol or alcohol for the reaction, but it requires the use of special pressure-resistant equipment because the above reaction is carried out under high-pressure conditions.

The alcoholysis method is a method of depolymerizing a polyester by heating it in an alcohol solvent. This method has the advantages that, for example, when polyethylene terephthalate (PET) is depolymerized using methanol as a solvent, dimethyl terephthalate is produced as a monomer by the depolymerization reaction, which is efficient, and the depolymerization reaction is also relatively fast. However, since the alcohol used as a solvent has a low boiling point and pressurization is necessary to advance the reaction, special pressure-resistant equipment is required.

The glycolysis method is a method of depolymerizing a polyester by heating it with a depolymerization catalyst such as sodium carbonate in an excess of an alkylene glycol solvent to produce bis(hydroxyalkyl) terephthalate and ethylene glycol. Although the glycolysis method can be reacted at normal pressure, it has a problem of a relatively long reaction time.

The method using microorganisms is a method of obtaining terephthalic acid by hydrolyzing polyethylene terephthalate with an enzyme, and although the hydrolysis reaction can proceed under mild reaction conditions, there is room for improvement in terms of efficiency.

In addition to these chemical recycling methods, a method has been proposed for producing a terephthalate salt by adding an alcohol and an ether to polyethylene terephthalate and reacting it with an equivalent or excess amount of an alkali, as a method that can decompose it into monomers within a short time under relatively mild conditions (Patent Literature 1, Non-Patent Literature 1, etc.).

**Citation List**

**Patent Literature**

Patent Literature 1: Japanese Unexamined Patent Publication No. H10-087529

**Non Patent Literature**

Non Patent Literature 1: Oku et. al., Polymer Journal, vol. 29, No. 9, 708-712 (1997)

**Summary of Invention**

**Technical Problem**

Patent Literature 1 describes that polyethylene terephthalate can be depolymerized by reacting it with sodium hydroxide to be decomposed into a terephthalate salt and ethylene glycol, and that the depolymerization reaction proceeds at 40 to 75°C. However, from the viewpoint of the reaction rate of depolymerization (that is, the conversion rate from polyethylene terephthalate to terephthalic acid), there was still room for improvement. In addition, since ethers may produce peroxides upon deterioration, there were concerns in industrially carrying out the depolymerization of polyester using ethers.

The present disclosure relates to a method for depolymerizing a polyester that exhibits an excellent conversion rate to a dicarboxylic acid (e.g., terephthalic acid) or a hydroxycarboxylic acid by depolymerization even under mild reaction conditions.

**Solution to Problem**

The present inventors prepared a composition comprising a predetermined alkali compound such as sodium hydroxide and an alcohol-based solvent and/or a ketone-based solvent, and when depolymerizing a polyester using the composition, they found that the conversion rate to terephthalic acid is greatly affected by the amount of solvent used. The present disclosure is based on these findings.

The present disclosure includes the following.

[1] A method for depolymerizing a polyester, the method comprising:

forming a mixture comprising the following components (a), (b), and (c):

(a) a polyester,
(b) one or more alkali compounds selected from the group consisting of an alkali metal hydroxide, an alkali metal carbonate, an alkali metal bicarbonate, an alkaline earth metal hydroxide, an alkaline earth metal carbonate, and an alkaline earth metal bicarbonate, and
(c) a solvent comprising at least one of an alcohol-based solvent or a ketone-based solvent,

and depolymerizing the polyester in the mixture,
wherein a temperature of the mixture at a time when the mixture comprising an entire amount of the polyester, the alkali compound, and the solvent is formed is 75°C or less, and
a ratio of an amount of the polyester to an amount of the solvent is **from 0.2** to 20 on a mass basis.

[2] The method according to [1], wherein the alkali compound is soluble in an amount of 100 g or more per 100 g of water at $20 \pm 5$°C.
[3] The method according to [1] or [2], wherein the alkali compound is one or more selected from the group consisting of an alkali metal hydroxide, an alkali metal carbonate, and an alkali metal bicarbonate.
[4] The method according to [1] or [2], wherein the alkali compound is an alkali metal hydroxide, an alkaline earth metal hydroxide, or both.
[5] The method according to any one of [1] to [4], wherein the alkali compound is one or more of an alkali metal hydroxide or an alkaline earth metal hydroxide, and a total molar amount of hydroxide ions in the alkali metal hydroxide and hydroxide ions in the alkaline earth metal hydroxide is in a range of 0.9 to 3.0 relative to a molar amount of the polyester based on repeating units before the mixture is formed.
[6] The method according to any one of [1] to [5], wherein a temperature of the mixture at a time when the mixture comprising an entire amount of the polyester, the alkali compound, and the solvent is formed is from 0°C to 75°C.
[7] The method according to any one of [1] to [6], wherein a temperature of the mixture at a time when the mixture comprising an entire amount of the polyester, the alkali compound, and the solvent is formed is from 35°C to 55°C.
[8] The method according to any one of [1] to [7], wherein a ratio of an amount of the polyester to an amount of the solvent is from 0.25 to 2.2 on a mass basis.
[9] The method according to any one of [1] to [8], wherein the polyester is polyethylene terephthalate.
[10] The method according to any one of claims [1] to [9], wherein the alkali compound is sodium hydroxide, potassium hydroxide, or both.
[11] The method according to any one of [1] to [10], wherein the alcohol-based solvent is a lower alcohol.
[12] The method according to any one of [1] to [11], wherein the mixture comprises particles comprising the polyester.
[13] The method according to [12], wherein the particles comprising the polyester are a granular material having an average particle size of from 150 to 350 $\mu$m.
[14] The method according to [12] or [13], wherein a crystallinity of the particles comprising the polyester is 50% or less.
[15] The method according to any one of [12] to [14], wherein the mixture is a powdery mixture comprising the particles comprising the polyester and the solvent integrated with the particles.
[16] The method according to any one of [1] to [15], wherein the polyester before the mixture is formed has an intrinsic viscosity of 0.8 dl/g or less.

[17] The method according to any one of [1] to [16], wherein the polyester is prepared from used bottle containers comprising polyester.

[18] A method for producing a diol and a dicarboxylic acid, the method comprising producing a diol and a dicarboxylate from a polyester by the method according to any one of [1] to [17].

[19] A method for recycling a polyester, comprising producing a polyester by polymerization of a polymerization raw material comprising at least one of the diol obtained by the method according to [18], or a dicarboxylic acid produced from the dicarboxylate.

[1]' A method for depolymerizing a polyester, characterized by comprising:

mixing the following components (a), (b), and (c):

(a) a polyester,
(b) one or more alkali metal compounds selected from the group consisting of an alkali metal hydroxide, an alkali metal carbonate, an alkali metal bicarbonate, an alkaline earth metal hydroxide, an alkaline earth metal carbonate, and an alkaline earth metal bicarbonate,
(c) a solvent comprising an alcohol-based solvent and/or a ketone-based solvent,

wherein the mixing is performed at 75°C or less, and
the components (a), (b), and (c) are mixed such that the component (a) is from 0.2 to 20 on a mass basis relative to the component (c).

[2]' The method according to [1]', wherein

the component (b) is an alkali metal hydroxide and/or an alkaline earth metal hydroxide, and
the molar amount of hydroxide ions of the alkali metal hydroxide and/or the alkaline earth metal hydroxide is in a range of 1.8 to 3.0 relative to a molar amount of the polyester based on repeating units added to the alcohol-based solvent and/or the ketone-based solvent.

[3]' The method according to [1]' or [2]', wherein the mixing is performed at 35 to 55°C.

[4]' The method according to any one of [1]' to [3]', wherein the component (a) is from 0.25 to 2.2 on a mass basis relative to the component (c).

[5]' The method according to any one of [1]' to [4]', wherein the (a) polyester is polyethylene terephthalate.

[6]' The method according to any one of [1]' to [5]', wherein the component (b) is sodium hydroxide or potassium hydroxide.

[7]' The method according to any one of [1]' to [6]', wherein the alcohol-based solvent is a lower alcohol.

[8]' The method according to any one of [1]' to [7]', wherein the (a) polyester is a granular material having an average particle size of from 150 to 350 $\mu$m.

[9]' The method according to any one of [1]' to [8]', wherein a crystallinity of the (a) polyester is 50% or less.

[10]' The method according to any one of [1]' to [9]', wherein the (a) polyester has an intrinsic viscosity of 0.8 dl/g or less.

[11]' The method according to any one of [1]' to [10]', wherein the (a) polyester is prepared from used bottle containers composed of polyester.

[12]' A method for producing a diol component and a dicarboxylic acid component by depolymerizing a polyester, wherein the production method is performed by the method according to any one of [1]' to [11]'.

[13]' A method for recycling a polyester, wherein a polyester is regenerated by using a product obtained by the method according to any one of [1]' to [11]' as at least a part of a polyester polymerization raw material.

## Advantageous Effects of Invention

According to the present disclosure, a method for depolymerizing a polyester is provided that exhibits an excellent conversion rate to a dicarboxylic acid such as terephthalic acid, or a hydroxycarboxylic acid by depolymerization even under mild reaction conditions. The method of the present disclosure can be used for industrial recycling of used polyester products.

## Description of Embodiments

<Method for Depolymerizing Polyester>

An example of a method for depolymerizing a polyester comprises mixing a polyester (hereinafter also referred to as "component (a)"), a predetermined alkali compound (hereinafter also referred to as "component (b)"), and a solvent comprising an alcohol-based solvent and/or a ketone-based solvent (hereinafter also referred to as "component (c)"). The alkali compound comprises at least one of an alkali metal compound or an alkaline earth metal compound. The solvent comprises at least one of an alcohol-based solvent or a ketone-based solvent. Mixing the polyester, the alkali compound, and the solvent includes first mixing them with each other to form a mixture, and stirring or kneading the formed mixture. The polyester is depolymerized in the formed mixture. A powder comprising particles comprising the polyester may be mixed with the alkali compound and the solvent. Hereinafter, an example of the method for depolymerizing a polyester according to the present disclosure will be described in detail.

The polyester is typically a polymer formed by polycondensation of one or more dicarboxylic acids and one or more diols. The alkali compound of component (b) reacts with the polyester of component (a) to produce a dicarboxylate and a diol. That is, a depolymerization reaction proceeds in which the polyester is decomposed into a dicarboxylate and a diol. Taking polyethylene terephthalate as an example, a terephthalate salt and ethylene glycol are produced. In this reaction (depolymerization reaction), the presence of a solvent comprising an alcohol-based solvent and/or a ketone-based solvent in a predetermined ratio relative to the amount of polyester allows the depolymerization reaction to proceed efficiently even under mild conditions such as 75°C or less. Specifically, in the mixture, the ratio of the amount of the polyester to the amount of the solvent of component (c) can be from 0.2 to 20 on a mass basis. That is, by mixing components (a), (b), and (c) such that the polyester is in a range of 0.2 to 20 on a mass basis relative to the alcohol-based solvent and/or ketone-based solvent of component (c), the polyester can be efficiently decomposed into a dicarboxylate and a diol even under mild conditions. The polyester may be a polymer formed by polycondensation of a hydroxycarboxylic acid compound having a hydroxy group and a carboxy group (for example, lactic acid). In that case, a salt of the hydroxycarboxylic acid compound can be formed by depolymerization of the polyester under mild conditions. In the salt of the hydroxycarboxylic acid compound, the carboxy group usually forms a salt. The polyester may comprise a dicarboxylic acid unit derived from a dicarboxylic acid, a diol unit derived from a diol, and a hydroxycarboxylic acid unit derived from a hydroxycarboxylic acid.

If the ratio of the amount of the polyester to the amount of the solvent (component (a) to component (c)) is less than 0.2 on a mass basis, the conversion rate to a dicarboxylate or the like tends to decrease under mild conditions. On the other hand, if the ratio of the amount of the polyester to the amount of the solvent (component (a) to component (c)) exceeds 20 on a mass basis, the amount of solvent is too small, making it difficult for the depolymerization reaction of the polyester to proceed uniformly, or not proceed, and on the contrary, the conversion rate to a dicarboxylate or the like tends to decrease. From the same viewpoint, the ratio of the amount of the polyester to the amount of the solvent may be 0.25 or more, 0.3 or more, or 0.35 or more, and may be 10 or less, 5.0 or less, 3.0 or less, 2.2 or less, 2.0 or less, or 1.0 or less, on a mass basis. The ratio of the amount of the polyester to the amount of the solvent may be 0.25 to 10, 0.3 to 10, 0.35 to 10, 0.25 to 5.0, 0.3 to 5.0, 0.35 to 5.0, 0.25 to 3.0, 0.3 to 3.0, 0.35 to 3.0, 0.25 to 2.2, 0.3 to 2.2, 0.35 to 2.2, 0.25 to 2.0, 0.3 to 2.0, 0.35 to 2.0, 0.25 to 1.0, 0.3 to 1.0, or 0.35 to 1.0 on a mass basis.

The alkali compound (component (b)) used in the present disclosure is not particularly limited as long as it is one or more selected from the group consisting of an alkali metal hydroxide, an alkali metal carbonate, an alkali metal bicarbonate, an alkaline earth metal hydroxide, an alkaline earth metal carbonate, and an alkaline earth metal bicarbonate.

Examples of the alkali metal hydroxide include lithium hydroxide, sodium hydroxide, potassium hydroxide, rubidium hydroxide, cesium hydroxide, and francium hydroxide. Examples of the alkali metal carbonate include lithium carbonate, sodium carbonate, potassium carbonate, rubidium carbonate, cesium carbonate, and francium carbonate. Examples of the alkali metal bicarbonate include lithium bicarbonate, sodium bicarbonate, potassium bicarbonate, rubidium bicarbonate, cesium bicarbonate, and francium bicarbonate. Examples of the alkaline earth metal hydroxide include beryllium hydroxide, magnesium hydroxide, calcium hydroxide, strontium hydroxide, barium hydroxide, and radium hydroxide. Examples of the alkaline earth metal carbonate include beryllium carbonate, magnesium carbonate, calcium carbonate, strontium carbonate, barium carbonate, and radium carbonate. Examples of the alkaline earth metal bicarbonate include beryllium bicarbonate, magnesium bicarbonate, calcium bicarbonate, strontium bicarbonate, barium bicarbonate, and radium bicarbonate.

The alkali compound may be one that readily dissolves in the solvent constituting the mixture. Since both alcohol-based solvents and ketone-based solvents are solvents with relatively high hydrophilicity, an alkali compound that shows higher solubility in water generally tends to show higher solubility in a solvent comprising at least one of an alcohol-based solvent or a ketone-based solvent. Therefore, an alkali compound that readily dissolves in a solvent comprising at least one of an alcohol-based solvent or a ketone-based solvent can be selected using the magnitude of solubility in water as an index. From this viewpoint, the alkali compound may be one that dissolves in an amount of 100 g or more per 100 g of water at $20 \pm 5$°C. Here, "100 g or more" means that the amount of the alkali compound that dissolves in 100 g of water at $20 \pm 5$°C is 100 g or more, and may also be described as 100 g/100 g-$H_2O$ or more. The same applies to other numerical ranges.

The amount of the alkali compound that dissolves in water at $20 \pm 5°C$ is 100 g/100 g-$H_2O$ or more, preferably 102 g/100 g-$H_2O$ or more, more preferably 104 g/100 g-$H_2O$ or more, and still more preferably 106 g/100 g-$H_2O$ or more. The upper limit of the amount of the alkali compound that dissolves in water at $20 \pm 5°C$ is not particularly limited, but may be, for example, 300 g/100 g-$H_2O$ or less. The amount of the alkali compound that dissolves in water at $20 \pm 5°C$ is 100 to 300 g/100 g-$H_2O$, preferably 102 to 300 g/100 g-$H_2O$, more preferably 104 to 300 g/100 g-$H_2O$, and still more preferably 106 to 300 g/100 g-$H_2O$.

The alkali compound may be one or more alkali metal compounds selected from the group consisting of an alkali metal hydroxide, an alkali metal carbonate, and an alkali metal bicarbonate. In particular, the alkali compound may be sodium hydroxide, potassium hydroxide, or both.

In an example of the present disclosure, the molar amount of hydroxide ions of the alkali metal hydroxide and/or the alkaline earth metal hydroxide contained in the mixture together with the solvent comprising an alcohol-based solvent and/or a ketone-based solvent, in other words, the total molar amount of hydroxide ions in the alkali metal hydroxide and hydroxide ions in the alkaline earth metal hydroxide, may be adjusted to be in a range of 1.8 to 3.0 relative to the molar amount of the polyester based on the repeating units to be added. "The amount of the polyester based on the repeating units " is the amount, expressed in moles, of the sum of the total amount of repeating units consisting of polycarboxylic acid units and diol units contained in the polyester, and the total amount of repeating units consisting of hydroxycarboxylic acid units contained in the polyester. When the molar amount of hydroxide ions of the alkali metal hydroxide and/or the alkaline earth metal hydroxide is 1.8 or more relative to the molar amount of the polyester based on the repeating units, the conversion rate to a carboxylate or a salt of a hydroxycarboxylic acid tends to easily reach 90% or more when all the hydroxide ions of the alkali metal hydroxide and/or the alkaline earth metal hydroxide are consumed in the depolymerization reaction. On the other hand, if the molar amount of hydroxide ions of the alkali metal hydroxide and/or the alkaline earth metal hydroxide is 3.0 or less relative to the molar amount of the polyester based on the repeating units, when the produced polyester salt (dicarboxylate or salt of a hydroxycarboxylic acid) is neutralized with an acid to be converted into a free form, the cost of chemicals for neutralizing the excess hydroxide ions of the alkali metal hydroxide and/or the alkaline earth metal hydroxide and the cost of disposing of the neutralized salt are reduced, which can be economically advantageous.

In another embodiment, when the molar amount of hydroxide ions of the alkali metal hydroxide and/or the alkaline earth metal hydroxide is 0.9 or more relative to the molar amount of the polyester based on the repeating units, the conversion rate to a carboxylate or a salt of a hydroxycarboxylic acid tends to easily reach 90% or more when all the hydroxide ions of the alkali metal hydroxide and/or the alkaline earth metal hydroxide are consumed in the depolymerization reaction. On the other hand, if the molar amount of hydroxide ions of the alkali metal hydroxide and/or the alkaline earth metal hydroxide is 1.5 or less relative to the molar amount of the polyester based on the repeating units, when the produced polyester salt (dicarboxylate or salt of a hydroxycarboxylic acid) is neutralized with an acid to be converted into a free form, the cost of chemicals for neutralizing the excess hydroxide ions of the alkali metal hydroxide and/or the alkaline earth metal hydroxide and the cost of disposing of the neutralized salt are reduced, which can be economically advantageous.

From the above, the molar amount of hydroxide ions of the alkali metal hydroxide and/or the alkaline earth metal hydroxide may be 0.7 to 5.0, preferably 0.9 to 3.0, and more preferably 1.8 to 3.0, relative to the molar amount of the polyester based on the repeating units. In another embodiment, the molar amount of hydroxide ions of the alkali metal hydroxide and/or the alkaline earth metal hydroxide is preferably 0.7 to 2.0, and more preferably 0.9 to 1.5.

The entire amount of the alkali compound may be dissolved in the solvent, or a part or all of the alkali compound may be dispersed as a solid in the mixture without being dissolved in the solvent. In particular, the alkali metal hydroxide and/or the alkaline earth metal hydroxide may be dissolved in the solvent comprising an alcohol-based solvent and/or a ketone-based solvent. The alkali metal hydroxide and/or the alkaline earth metal hydroxide may be dispersed as a solid that does not dissolve in the solvent.

By setting the concentrations of the above-mentioned component (a) polyester, component (c) solvent comprising an alcohol-based solvent and/or a ketone-based solvent, and component (b) alkali compound within the ranges as described above, the polyester can be efficiently decomposed into a dicarboxylate, a diol, and the like even under mild reaction conditions such as 75°C or less. The depolymerization reaction can be carried out at a temperature lower than the boiling point of the alcohol-based solvent (or ketone-based solvent) used, for example, at 0 to 75°C, 1 to 75°C, 10 to 75°C, 15 to 75°C, 20 to 70°C, or 35 to 55°C. In other words, the temperature of the mixture at the time when the mixture comprising the entire amount of the polyester, the alkali compound, and the solvent is first formed by mixing them may be 75°C or less, 0 to 75°C, 1 to 75°C, 10 to 75°C, 20 to 70°C, or 35 to 55°C. The depolymerization reaction can proceed even at a temperature exceeding 75°C, but in that case, the solvent used tends to volatilize. Although volatilization of the solvent can be suppressed by carrying out the depolymerization reaction under pressurized conditions even at high temperatures, low temperature conditions do not necessarily require complex equipment for pressurization, which is advantageous from an industrial viewpoint. After the mixture is formed, the temperature of the mixture while stirring or kneading the mixture may be within the above range. However, the temperature of the mixture may exceed 75°C due to reaction heat or the like.

The temperature of the mixture while stirring or kneading the mixture may be equal to or lower than the boiling point of the solvent. The mixture may be stirred or kneaded in an environment at room temperature (5 to 45°C) without heating the mixture.

The alcohol-based solvent to be used is not particularly limited, and examples thereof include methanol, ethanol, propanol, isopropanol, butanol, and benzyl alcohol. The alcohol-based solvent may be a lower alcohol. The lower alcohol can be an alkyl alcohol having 1 to 5 carbon atoms. The alcohol-based solvent may comprise butanol, propanol, isopropanol, ethanol, methanol, or a combination of two or more selected therefrom, and in particular may comprise ethanol, methanol, or a combination thereof. The alcohol-based solvent may be one type or two or more types. The alcohol-based solvent mixed with the polyester and the alkali compound may contain a trace amount of water.

The ketone-based solvent to be used is not particularly limited, and examples thereof include acetone, methyl ethyl ketone, diethyl ketone, methyl propyl ketone, methyl isobutyl ketone, methyl amyl ketone, cyclohexanone, isophorone, acetophenone, and benzophenone. The ketone-based solvent may be one type or two or more types. The ketone-based solvent mixed with the polyester and the alkali compound may contain a trace amount of water.

In an example of the present disclosure, the solvent comprises an alcohol-based solvent, particularly methanol or ethanol. However, the solvent contained in the mixture together with the predetermined alkali compound described above may comprise other solvents other than the alcohol-based solvent and/or the ketone-based solvent. For example, the solvent may comprise water. In this case, the ratio of the amount of the polyester to the mass of the entire solvent, which also comprises other solvents other than the alcohol-based solvent and/or the ketone-based solvent, can be from 0.2 to 20 on a mass basis. If the composition containing the alkali compound to be mixed with the polyester, or the solvent, contains a solvent capable of dissolving a dicarboxylate or a salt of a hydroxycarboxylic acid, such as water, the depolymerization reaction rate tends to slow down, and as a result, the conversion rate from the polyester to the dicarboxylate or the salt of the hydroxycarboxylic acid may be relatively reduced. Therefore, in the solvent contained in the mixture together with the alkali compound, the proportion of the total amount of the alcohol-based solvent and the ketone-based solvent may be 50% by mass or more, 70% by mass or more, or 90% by mass or more, based on the total amount of the solvent, and may be 100% by mass or less. In the solvent contained in the mixture together with the alkali compound, the proportion of the total amount of the alcohol-based solvent and the ketone-based solvent may be 50 to 100% by mass, 70 to 100% by mass, or 90 to 100% by mass, based on the total amount of the solvent.

The polyester applicable to the depolymerization method of the present disclosure may be a polyester obtained by polycondensing a diol component (diol) and a dicarboxylic acid component (dicarboxylic acid), or may be a polyester obtained by polycondensing a compound having both a hydroxy group and a carboxy group in the molecule. Examples of the polyester include aromatic polyesters such as polyethylene terephthalate, polytrimethylene terephthalate, polybutylene terephthalate, polyethylene naphthalate, and polyethylene furanoate; fully aromatic polyesters such as polyarylate and liquid crystal polymers; polycarbonate esters such as polycarbonate; aliphatic polyesters such as polylactic acid-based polymers, polybutylene succinate-based polymers, polyethylene adipate-based polymers, polyethylene succinate-based polymers, polycaprolactone-based polymers, and polyhydroxyalkanoate-based polymers; and aliphatic-aromatic polyesters such as polybutylene adipate terephthalate, polyethylene adipate terephthalate, and polyethylene succinate terephthalate. Among these, the application of polyethylene terephthalate, which is used in large quantities as a material for bottle containers, is particularly useful.

Polyethylene terephthalate is obtained by polycondensing two components, ethylene glycol and terephthalic acid, as main constituent monomers. The polyethylene terephthalate may comprise other monomers as copolymerization components, in addition to these two components, as a diol component or a dicarboxylic acid component.

Examples of the dicarboxylic acid component as a copolymerization component of polyethylene terephthalate include malonic acid, succinic acid, glutaric acid, adipic acid, suberic acid, sebacic acid, dodecanedioic acid, eicosanedioic acid, pimelic acid, azelaic acid, methylmalonic acid and ethylmalonic acid, adamantanedicarboxylic acid, norbornenedicarboxylic acid, cyclohexanedicarboxylic acid, decalindicarboxylic acid, isophthalic acid, phthalic acid, 1,4-naphthalenedicarboxylic acid, 1,5-naphthalenedicarboxylic acid, 2,6-naphthalenedicarboxylic acid, 1,8-naphthalenedicarboxylic acid, 4,4'-diphenyldicarboxylic acid, 4,4'-diphenyletherdicarboxylic acid, 5-sodium sulfoisophthalic acid, phenylenedanedicarboxylic acid, anthracenedicarboxylic acid, phenanthrenedicarboxylic acid, 9,9'-bis(4-carboxyphenyl)fluorenic acid, 2,5-furandicarboxylic acid, and their ester derivatives.

Examples of the diol component as a copolymerization component of polyethylene terephthalate include 1,2-propanediol, 1,3-propanediol, butanediol, 2-methyl-1,3-propanediol, hexanediol, neopentyl glycol, cyclohexanedimethanol, cyclohexanediethanol, decahydronaphthalenedimethanol, decahydronaphthalenediethanol, norbornanedimethanol, norbornanediethanol, tricyclodecanedimethanol, tricyclodecaneethanol, tetracyclododecanedimethanol, tetracyclododecanediethanol, decalindimethanol, decalindiethanol, 5-methylol-5-ethyl-2-(1,1-dimethyl-2-hydroxyethyl)-1,3-dioxane, cyclohexanediol, bicyclohexyl-4,4'-diol, 2,2-bis(4-hydroxycyclohexylpropane), 2,2-bis(4-(2-hydroxyethoxy)cyclohexyl)propane, cyclopentanediol, 3-methyl-1,2-cyclopentadiol, 4-cyclopentene-1,3-diol, adamantanediol, p-xylene glycol, bisphenol A, bisphenol S, styrene glycol, trimethylolpropane, pentaerythritol, and bis-β-hydroxyethyl terephthalate (BHET).

It is known that polyethylene terephthalate, which is a raw material for PET bottles, is a partially crystalline polymer having both an amorphous part and a crystalline part. The polyester may comprise a copolymerization component that reduces crystallinity. For example, polyethylene terephthalate comprising copolymerization components such as isophthalic acid and adipic acid as dicarboxylic acid components has a relatively low crystallinity and is excellent in the conversion rate to a dicarboxylate upon depolymerization.

The crystallinity of the polyester subjected to depolymerization is not limited, but may be 50% or less. The crystallinity of the particles comprising the polyester may be 50% or less. From the viewpoint of the conversion efficiency from the polyester to a dicarboxylate or a hydroxycarboxylic acid, a lower crystallinity of the polyester subjected to depolymerization is advantageous, but according to the method of the present disclosure, even a polyester with a crystallinity of about 50% (crystallized polyester) can be efficiently converted to a dicarboxylate or a hydroxycarboxylic acid under mild conditions. The lower the crystallinity of the polyester, the more easily the depolymerization reaction proceeds, and therefore, the conversion efficiency to a dicarboxylate or a hydroxycarboxylic acid can be particularly improved. The crystallinity of the polyester (particles comprising the polyester) may be 40% or less, or 20% or less. The crystallinity can be adjusted, as described above, by adding a copolymer monomer to the polyester, and by a stretching orientation treatment or the like.

The crystallinity means the crystallinity obtained from wide-angle X-ray diffraction measurement, and specifically, can be calculated from the wide-angle X-ray diffraction measurement profile by the following formula:

Crystallinity (%) = {Integrated value of crystalline peaks / (Integrated value of crystalline peaks + Integrated value of amorphous peaks)} $\times$ 100.

The intrinsic viscosity (IV) of the polyester may be 0.8 dl/g or less. A smaller intrinsic viscosity tends to indicate a smaller molecular weight of the polyester, which can contribute to promoting a more efficient depolymerization reaction. Even if the intrinsic viscosity of the polyester constituting a used polyester product (e.g., a bottle container) is high, the intrinsic viscosity of flakes or the like formed after re-melting the polyester for recycling can be reduced. There is no particular lower limit for the intrinsic viscosity, but about 0.3 dl/g can be the lower limit of the intrinsic viscosity. The intrinsic viscosity of the polyester may be 0.3 to 0.8 dl/g.

The shape of the polyester may be a powder composed of particles comprising the polyester. According to the Dictionary of Powder Engineering Terms, the basic physical properties of a powder are characterized by particle shape and particle size. The particles constituting the polyester powder can take various geometric shapes produced by various processes, such as cubical, granular, blocky, platy, flaky, film-like (thin powder particles, thinner than flaky), prismoidal, rodlike, acicular (needle-like), fibrous, dendritic, sponge, angular, sharp-edged (sharp-cornered), modular (rounded), irregular, or crystalline. The shape of the particles is based on the mechanical and crystallographic properties of the substance constituting the particles, and also depends on the production process (http://www.sptj.jp/powderpedia/words/12474/). In the present disclosure, the particles comprising the polyester can have any shape including the above shapes. The particles comprising the polyester may be flaky, fibrous, film-like, or granular, may be flaky or granular, and may be granular. That is, the mixture may comprise a granular material comprising the polyester.

The average particle size of the granular material comprising the polyester is not particularly limited, but may be 150 to 350 $\mu$m, or 200 to 300 $\mu$m. Here, the average particle size means the 50% particle size (D50: median diameter) when the particle size distribution measured by the laser diffraction scattering method is expressed as a cumulative distribution. The maximum particle size of the granular material comprising the polyester may be 200 to 1000 $\mu$m, 250 to 500 $\mu$m, or 250 to 400 $\mu$m.

The longest side of the flake-like particles comprising the polyester may be, for example, 1 to 50 mm, 1 to 20 mm, or 5 to 15 mm. Those obtained by collecting and shredding used PET bottles often have a flake shape, but the flake-like particles are not limited to those recovered from used PET bottles.

The longest side (length of the fibrous particles) of the fibrous particles comprising the polyester may be 1 to 500 mm, or 1 to 200 mm. The fibrous particles may be short fibers having a longest side (length) of about 1 to 100 mm. The fibrous polyester may be PET fibers used in clothing, but the fibrous polyester is not limited to these.

The longest side of the film-like particles comprising the polyester may be, for example, about 1 to 200 mm, 1 to 100 mm, or 1 to 50 mm.

In the present disclosure, components (a), (b), and (c) are mixed such that the blending ratio of each component is within the range as described above. In other words, a mixture is formed by mixing components (a), (b), and (c) such that the blending ratio of each component is within the range as described above, and the mixture is stirred or kneaded. The mixture may be a dispersion comprising a solution comprising the alkali compound and the solvent, and particles comprising the polyester dispersed in the solution. Alternatively, the mixture may be a powdery mixture comprising particles comprising the polyester and the solvent integrated with the particles. The powdery mixture may comprise a solid alkali compound. The solid alkali compound may be integrated with the particles comprising the polyester, or may form particles separate from the particles comprising the polyester. The solvent and/or the alkali compound integrated with the particles comprising the polyester may be attached to the surface of the particles comprising the polyester, or may have penetrated into the interior of the particles comprising the polyester. In the powdery mixture, the depolymerization of the polyester can proceed particularly efficiently.

According to an example of the present disclosure, the depolymerization reaction of the polyester proceeds even by mixing the components under mild conditions such as 75°C or less. From the viewpoint of the conversion rate to a dicarboxylate or a salt of a hydroxycarboxylic acid, the mixing (stirring or kneading) may be carried out for about 50 minutes, or about 30 minutes. Thereby, the conversion rate to a dicarboxylate or a salt of a hydroxycarboxylic acid tends to be 50% or more. Mixing means an operation of stirring or kneading in a container so that the composition containing the alkali compound (composition comprising the alkali compound and the solvent) and the polyester have uniform properties. Specifically, mixing means performing stirring or kneading of the mixture containing these components. The speed of stirring and kneading is not limited. From the viewpoint of work safety and equipment capacity, the stirring speed (for example, the rotation speed of the stirring blade) may be, for example, 250 to 1500 rpm, or 500 to 1000 rpm. The kneading speed (for example, the rotation speed of the kneading blade) may be, for example, 5 to 150 rpm, or 10 to 100 rpm.

<Method for Recycling Polyester>

In another example of the present disclosure, a recycling method is provided in which a polyester is regenerated by using a product produced by the above-described method for depolymerizing a polyester as at least a part of a polyester polymerization raw material. That is, by applying the method for depolymerizing a polyester, a dicarboxylate and a dialcohol are obtained as products from a polyester prepared from a used polyester product (for example, a used bottle container comprising polyester). The dicarboxylate can be separated as a solid. The dicarboxylate is usually converted to a dicarboxylic acid before being used as a polyester polymerization raw material. The dialcohol can be separated from a mixture with the alcohol-based solvent and/or the ketone-based solvent by a known operation such as fractional distillation. From a polyester containing hydroxycarboxylic acid units, a product containing a salt of a hydroxycarboxylic acid is obtained. The salt of the hydroxycarboxylic acid is usually converted to a hydroxycarboxylic acid before being used as a polyester polymerization raw material.

In an example of the present disclosure, polyethylene terephthalate is used, methanol is used as the solvent comprising an alcohol-based solvent and/or a ketone-based solvent, and sodium hydroxide is used as the alkali metal hydroxide. In that case, by depolymerization of the polyethylene terephthalate, sodium terephthalate is obtained as a solid, and ethylene glycol can be obtained by a fractional distillation operation of a mixture of ethylene glycol and methanol. The obtained sodium terephthalate can be converted to terephthalic acid by the addition of an acid. Polyethylene terephthalate can be regenerated from the terephthalic acid and ethylene glycol thus obtained. It is also possible to dissolve the mixture of sodium terephthalate, ethylene glycol, and methanol obtained after depolymerization in water to form an aqueous solution containing sodium terephthalate, ethylene glycol, and methanol, and then crystallize and recover terephthalic acid by the addition of an acid. At this time, the methanol and ethylene glycol remaining in the aqueous solution can be recovered respectively by a fractional distillation operation. Polyethylene terephthalate can be regenerated or produced from the terephthalic acid and ethylene glycol thus obtained. The terephthalic acid and ethylene glycol obtained by the method of the present disclosure may be used as part of the diol component and dicarboxylic acid component used when producing the polyester.

**Examples**

Next, the present invention will be described in more detail with reference to Examples. However, the present invention is not limited to these Examples.

<Preparation of Samples>

(1) Polyester

- PET Sample 1

A commercially available amorphous type PET resin (PBK-1, intrinsic viscosity 0.65 dl/g, manufactured by Bell Polyester Products, Inc.) was prepared. This PET resin was freeze-pulverized to obtain PET Sample 1, which is a granular material comprising polyethylene terephthalate.

The particle size distribution of PET Sample 1 was measured by a wet method using a laser diffraction particle size distribution analyzer (Anton Paar, PSA1190) using a scattering method. From the obtained particle size distribution, the 50% particle size (D50 median diameter) when expressed as a cumulative distribution was calculated, and this value was taken as the average particle size. The average particle size of PET Sample 1 was 218 μm.

Wide-angle X-ray diffraction measurement of PET Sample 1 was performed using a wide-angle X-ray diffractometer (RINT2500HL, manufactured by Rigaku). The conditions for the wide-angle X-ray diffraction measurement were: X-ray source CuKα radiation ($\lambda = 0.1542$ nm), output 50 kV-250 mA, scanning speed 0.5 deg/min, slit width DS 0.6 mm + 10 mmH. The range of $2\theta = 1.5$ to $70°$ of the obtained diffraction pattern was subjected to peak fitting, the integrated intensities of the angular peaks were summed for the crystalline component and the amorphous component, respectively, and these values were taken as the crystalline component integrated intensity I and the amorphous component integrated intensity Ia. From I and Ia, the crystallinity X was determined according to the following formula.

$$X = \{I / (I + Ia)\} \times 100 \ (\%)$$

X: Crystallinity
I: Crystalline component integrated intensity
Ia: Amorphous component integrated intensity

The crystallinity of PET Sample 1 determined as described above was 0.40%.

- PET Sample 2

A commercially available crystalline type PET resin (TK-3, intrinsic viscosity 0.65 dl/g, manufactured by Bell Polyester Products, Inc.) was prepared. This PET resin was freeze-pulverized to obtain PET Sample 2, which is a granular material comprising polyethylene terephthalate.

The average particle size of PET Sample 2 was calculated in the same manner as above and was found to be 268 μm. The crystallinity of PET Sample 2 was determined in the same manner as above and was found to be 22.8%.

[Examples 1-2, Comparative Examples 1-2]

A solution was prepared by dissolving potassium hydroxide in ethanol. After adjusting the temperature of this solution to 40°C, PET Sample 1 was added to the solution to form a mixture, which is a dispersion comprising PET Sample 1, potassium hydroxide, and ethanol in the masses shown in Table 1. After forming the mixture, the mixture was stirred for 10 minutes at a rotation speed of 760 rpm with a mechanical stirrer (BL600, manufactured by Shinto Scientific Co., Ltd.) without heating. The mixture was stirred under four conditions with the amount of PET Sample 1 added varying from 5 g (26 mmol) to 30 g (156 mmol) as shown in Table 1. Under the four conditions, the molar amount of potassium hydroxide in the solution was adjusted to be 2.2 relative to the molar amount of PET based on the repeating units. The depolymerization rate and terephthalic acid conversion rate calculated as described below were as shown in Table 1. As is clear from Table 1, it can be seen that as the amount of PET Sample 1 added increases on a mass basis relative to the reaction solvent, the depolymerization efficiency increases and a high effect is exhibited. The "mole number of repeating units" in the table is the molar amount of the repeating units ($C_{10}H_8O_4$) consisting of terephthalic acid units and ethylene glycol units contained in the PET sample. The initial temperature in the table is the temperature of the mixture at the time when the entire amount of the PET sample was added to the solution, that is, at the time when the mixture comprising the entire amount of the solution and the PET sample was formed.

<Depolymerization Rate>

After 10 minutes from the start of stirring the mixture, a sufficient amount of water was added to the mixture (reaction liquid) to dissolve the product. Unreacted PET Sample 1 was recovered by centrifugation. The recovered unreacted PET Sample 1 was dried by heating in a 100°C oven for 12 hours or more, and its mass $W_{fin}$ was measured.

The depolymerization rate was calculated using the following calculation formula.

$$\text{Depolymerization rate} \ (\%) = [(W_{int} - W_{fin}) / W_{int}] \times 100$$

In the above formula, $W_{int}$ represents the mass (g) of PET Sample 1 subjected to the reaction, and $W_{fin}$ represents the mass (g) of unreacted PET Sample 1 recovered after the reaction.

<Terephthalic Acid Conversion Rate>

The centrifugal supernatant from the centrifugation for recovering unreacted PET Sample 1 was placed in a volumetric flask, and water was added thereto to a total volume of 1 L. A portion of the solution in the volumetric flask was diluted 100-fold with a diluent (1N HCl aqueous solution:isopropanol = 1:9 (v/v)). Using the diluted solution as a sample, the quantification of terephthalic acid was performed by HPLC. The HPLC measurement conditions were as follows.

Column: L-column 2 ODS 2.1 mm × 150 mm, particle size 2 $\mu$m (Chemicals Evaluation and Research Institute, Japan)
Column temperature: 35°C
Mobile phase A: 0.1% acetic acid-containing water
Mobile phase B: 0.1% acetic acid-containing acetonitrile
Flow rate: 0.2 mL/min
Linear gradient: 15% B (0 min) → 80% B (26 min)
Detector: Photodiode array (wavelength 190-600 nm)

Based on the quantification result by HPLC, the terephthalic acid conversion rate was calculated from the following formula.

$$\text{Conversion rate } (\%) = [(W_{tpa} / 166) / (W_{int} / 192)] \times 100$$

In the above formula, $W_{int}$ is the mass (g) of PET Sample 1 subjected to the reaction, $W_{tpa}$ is the production amount (g) of terephthalic acid in terms of free form calculated based on the quantification result by HPLC, 166 is the molecular weight of terephthalic acid, and 192 is the formula weight of the repeating unit ($C_{10}H_8O_4$) of PET.

[Table 1]

| | (a) PET sample 1 | (c) Solvent | | (b) Alkali compound | | Ratio of PET mass to solvent mass (a)/(c) | Depolymerization | | Depolymerization rate (%) | Terephthalic acid conversion rate (%) |
| | Mass (mol of repeating unit) | Type | Mass (Volume) | Type | Mass (Molar amount) | | Initial temp. | Time | | |
|---|---|---|---|---|---|---|---|---|---|---|
| Ex. 1 | 20 g (104 mmol) | Ethanol | 78.9 g (100 mL) | KOH | 12.8 g (229 mmol) | 0.253 | 40°C | 10 min | 55.1 | 52.5 |
| Ex. 2 | 30 g (156 mmol) | Ethanol | 78.9 g (100 mL) | KOH | 19.3 g (343 mmol) | 0.38 | 40°C | 10 min | 93.1 | 89.4 |
| Comp. Ex. 1 | 5 g (26 mmol) | Ethanol | 78.9 g (100 mL) | KOH | 3.21 g (57 mmol) | 0.063 | 40°C | 10 min | 20.2 | 17.8 |
| Comp. Ex. 2 | 10 g (52 mmol) | Ethanol | 78.9 g (100 mL) | KOH | 6.41 g (114 mmol) | 0.127 | 40°C | 10 min | 35.2 | 33.0 |

[Examples 3-4, Comparative Examples 3-4]

After adjusting the temperature of a solution in which potassium hydroxide was added to ethanol to 50°C, PET Sample 2 was added to the solution to form a mixture, which is a dispersion comprising PET Sample 2, potassium hydroxide, and ethanol in the masses shown in Table 2. Experiments were conducted in the same manner as in Examples 1-2 and Comparative Examples 1-2, except that the formed mixture was stirred, and the depolymerization rate and terephthalic acid conversion rate were calculated. The obtained results were as shown in Table 2. As is also clear from Table 2, it can be seen that as the amount of PET Sample 2 added increases on a mass basis relative to the reaction solvent, the depolymerization efficiency increases and a high effect is exhibited.

[Table 2]

| | (a) PET sample 2 | (c) Solvent | | (b) Alkali compound | | Ratio of PET mass to solvent mass (a)/(c) | Depolymerization | | Depolymerization rate (%) | Terephthalic acid conversion rate (%) |
|---|---|---|---|---|---|---|---|---|---|---|
| | Mass (mol of repeating unit) | Type | Mass (Volume) | Type | Mass (Molar amount) | | Initial temp. | Time | | |
| Ex. 3 | 20 g (104 mmol) | Ethanol | 78.9 g (100 mL) | KOH | 12.8 g (229 mmol) | 0.253 | 50°C | 10 min | 48.8 | 45.4 |
| Ex. 4 | 30 g (156 mmol) | Ethanol | 78.9 g (100 mL) | KOH | 19.3 g (343 mmol) | 0.38 | 50°C | 10 min | 79.8 | 77.3 |
| Comp. Ex. 3 | 5 g (26 mmol) | Ethanol | 78.9 g (100 mL) | KOH | 3.21 g (57 mmol) | 0.063 | 50°C | 10 min | 27.4 | 24.5 |
| Comp. Ex. 4 | 10 g (52 mmol) | Ethanol | 78.9 g (100 mL) | KOH | 6.41 g (114 mmol) | 0.127 | 50°C | 10 min | 36.3 | 33.0 |

[Example 5]

A solution was prepared by dissolving sodium hydroxide in methanol. After adjusting the temperature of this solution to 40°C, 30 g of PET Sample 1 was added to the solution to form a mixture, which is a dispersion comprising PET Sample 1, sodium hydroxide, and methanol in the masses shown in Table 3. An experiment was conducted in the same manner as in Example 1, except that the formed mixture was stirred, and the depolymerization rate and terephthalic acid conversion rate were calculated. The obtained results were as shown in Table 3. For comparison, Example 1 is also shown in Table 3.

[Table 3]

| | (a) PET sample 1 | (c) Solvent | | (b) Alkali compound | | Ratio of PET mass to solvent mass (a)/(c) | Depolymerization | | Depolymerization rate (%) | Terephthalic acid conversion rate (%) |
|---|---|---|---|---|---|---|---|---|---|---|
| | Mass (mol of repeating unit) | Type | Mass (Volume) | Type | Mass (Molar amount) | | Initial temp. | Time | | |
| Ex. 5 | 30 g (156 mmol) | Methanol | 79.2 g (100 mL) | NaOH | 13.7 g (343 mmol) | 0.379 | 40°C | 10 min | 83.4 | 79.0 |
| Ex. 1 | 20 g (104 mmol) | Ethanol | 78.9 g (100 mL) | KOH | 12.8 g (229 mmol) | 0.253 | 40°C | 10 min | *55.1* | 52.5 |

[Example 6]

After adjusting the temperature of a solution in which sodium hydroxide was added to a mixed solvent of methanol/water (methanol:water (on a mass basis) = approx. 7:3) to 40°C, PET Sample 1 was added to the solution to form a mixture, which is a dispersion comprising PET Sample 1, sodium hydroxide, and the mixed solvent (methanol/water) in the masses shown in Table 4. An experiment was conducted in the same manner as in Example 1, except that the formed mixture was stirred, and the depolymerization rate and terephthalic acid conversion rate were calculated. The obtained results were as shown in Table 4.

[Example 7]

After adjusting the temperature of a solution in which sodium hydroxide was added to a mixed solvent of methanol/water (methanol:water (on a mass basis) = approx. 7:3) to 50°C, PET Sample 2 was added to the solution to form a mixture, which is a dispersion comprising PET Sample 2, sodium hydroxide, and the mixed solvent (methanol/water) in the masses shown in Table 4. An experiment was conducted in the same manner as in Example 3, except that the formed mixture was stirred, and the depolymerization rate and terephthalic acid conversion rate were calculated. The obtained results were as shown in Table 4.

[Comparative Example 5]

After adjusting the temperature of a solution in which sodium hydroxide was added to water to 40°C, PET Sample 1 was added to the solution to form a mixture, which is a dispersion comprising PET Sample 1, sodium hydroxide, and water in the masses shown in Table 4. An experiment was conducted in the same manner as in Example 6, except that the formed mixture was stirred, and the depolymerization rate and terephthalic acid conversion rate were calculated. The obtained results were as shown in Table 4.

As is also clear from Table 4, under the conditions of Examples 6 and 7 using a mixed solvent containing methanol:water in a ratio of approx. 7:3 on a mass basis, a high depolymerization rate of 70% or more was shown. In contrast, in Comparative Example 5, which used only water as the solvent, the depolymerization rate was a low value of less than 3%, even though the amount of PET added was a high value of 0.613 on a mass basis relative to the reaction solvent. This result suggested that it is advantageous for the solvent to comprise an alcohol-based solvent in order to increase the depolymerization efficiency.

[Table 4]

| | (a) PET sample | | (c) Solvent | (b) Alkali compound | | Ratio of PET mass to solvent mass (a)/(c) | Depolymerization | | Depolymerization rate (%) | Terephthalic acid conversion rate (%) |
|---|---|---|---|---|---|---|---|---|---|---|
| | | Mass (mol of repeating unit) | Type Mass (Volume) | Type | Mass (Molar amount) | | Initial temp. | Time | | |
| Ex. 6 | PET sample 1 | 50 g (260 mmol) | Methanol 56.8 g /Water 24.8 g (100 mL) | NaOH | 22.9 g (572 mmol) | 0.613 | 40°C | 10 min | 75.1 | 69.9 |
| Ex. 7 | PET sample 2 | 50 g (260 mmol) | Methanol 56.8 g /Water 24.8 g (100 mL) | NaOH | 22.9 g (572 mmol) | 0.613 | 50°C | 10 min | 71.7 | 68 |
| Comp. Ex. 5 | PET sample 1 | 50 g (260 mmol) | Water 81.6 g (81.6 mL) | NaOH | 22.9 g (572 mmol) | 0.613 | 40°C | 10 min | 2.9 | 4.2 |

[Example 8]

As PET Sample 3, a commercially available crystalline type PET resin powder (granular material, ES30-PD-000132, intrinsic viscosity 0.80 dl/g, maximum particle size 300 µm, manufactured by Goodfellow) was prepared. The crystallinity of PET Sample 3 was determined in the same manner as for PET Sample 1 and was found to be 39.5%.

150 g of methanol was heated to 50°C in a kneader (HIVIS DISPER MIX 3D-2 model, manufactured by PRIMIX Corporation). To this, 137 g (3.43 mol) of sodium hydroxide and 300 g of PET Sample 3 (molar amount based on repeating units: 1.56 mol) were added to form a powdery mixture comprising PET Sample 3, sodium hydroxide, and methanol in the masses shown in Table 5. The formed powdery mixture was kneaded for 15 minutes at a rotation speed of 40 rpm without heating. The depolymerization rate and terephthalic acid conversion rate were as shown in Table 5.

[Table 5]

| | (a) PET sample 3 | (c) Solvent | | (b) Alkali compound | | Ratio of PET mass to solvent mass (a)/(c) | Depolymerization | | Depolymerization rate (%) | Terephthalic acid conversion rate (%) |
|---|---|---|---|---|---|---|---|---|---|---|
| | Mass (mol of repeating unit) | Type | Mass (Volume) | Type | Mass (Molar amount) | | Initial temp. | Time | | |
| Ex. 8 | 300 g (1.56 mol) | Methanol | 150 g (189 mL) | NaOH | 137 g (3.43 mol) | 2 | 50°C | 15 min | 95.5 | 92.9 |

[Example 9]

PET Sample 4, formed by flaking recovered PET bottles, was prepared. PET Sample 4 was a powder comprising flake-like particles having a side size of 0.5 to 1 cm. The crystallinity of a sample obtained by freeze-pulverizing PET Sample 4 was measured in the same manner as for PET Sample 1 and was found to be 8.8%.

The flake-like PET Sample 4 was directly subjected to a depolymerization test. 150 g of methanol was placed in a desktop kneader (PNV-1, manufactured by IRIE SHOKAI Co., Ltd.) and heated to 50°C. To this, 137 g (3.43 mol) of sodium hydroxide and 300 g of PET Sample 4 (molar amount based on repeating units: 1.56 mol) were added to form a powdery mixture comprising PET Sample 4, sodium hydroxide, and methanol in the masses shown in Table 6. The formed powdery mixture was kneaded for 30 minutes at a rotation speed of 40 rpm without heating. The depolymerization rate and terephthalic acid conversion rate were as shown in Table 6.

[Table 6]

| | (a) PET sample 4 | (c) Solvent | | (b) Alkali compound | | Ratio of PET mass to solvent mass (a)/(c) | Depolymerization | | Depolymerization rate (%) | Terephthalic acid conversion rate (%) |
| | Mass (mol of repeating unit) | Type | Mass (Volume) | Type | Mass (Molar amount) | | Initial temp. | Time | | |
| Ex. 9 | 300 g (1.56 mol) | Methanol | 150 g (189 mL) | NaOH | 137 g (3.43 mol) | 2 | 50°C | 30 min | 63.7 | 68.9 |

[Example 10]

155 g of methanol was placed in a desktop kneader (PNV-1, manufactured by IRIE SHOKAI Co., Ltd.) and heated to 50°C. To this, 114 g (2.85 mol) of sodium hydroxide and 250 g of PET Sample 3 (molar amount based on repeating units: 1.30 mol) were added to form a powdery mixture comprising PET Sample 4, sodium hydroxide, and methanol in the masses shown in Table 7. The formed powdery mixture was kneaded for 30 minutes at a rotation speed of 50 rpm without heating. The depolymerization rate and terephthalic acid conversion rate were as shown in Table 7.

[Example 11]

155 g of methanol was placed in a desktop kneader (PNV-1, manufactured by IRIE SHOKAI Co., Ltd.). To the methanol at room temperature (21°C) in the kneader, 114 g (2.85 mol) of sodium hydroxide and 250 g of PET Sample 3 (molar amount based on repeating units: 1.30 mol) were added to form a powdery mixture comprising PET Sample 4, sodium hydroxide, and methanol in the masses shown in Table 7. The formed powdery mixture was kneaded for 30 minutes at a rotation speed of 50 rpm without heating. The depolymerization rate and terephthalic acid conversion rate were as shown in Table 7.

[Example 12]

125 g of methanol was placed in a desktop kneader (PNV-1, manufactured by IRIE SHOKAI Co., Ltd.). To the methanol at room temperature (19°C) in the kneader, 114 g (2.85 mol) of sodium hydroxide and 250 g of PET Sample 3 (molar amount based on repeating units: 1.30 mol) were added to form a powdery mixture comprising PET Sample 3, sodium hydroxide, and methanol in the masses shown in Table 7. The formed powdery mixture was kneaded for 8 minutes at 50 rpm without heating. The depolymerization rate and terephthalic acid conversion rate were as shown in Table 7.

[Example 13]

125 g of ethanol was placed in a desktop kneader (PNV-1, manufactured by IRIE SHOKAI Co., Ltd.). To the ethanol at room temperature (15°C) in the kneader, 114 g (2.85 mol) of sodium hydroxide and 250 g of PET Sample 3 (molar amount based on repeating units: 1.30 mol) were added to form a powdery mixture comprising PET Sample 3, sodium hydroxide, and ethanol in the masses shown in Table 7. The formed powdery mixture was kneaded for 8 minutes at 50 rpm without heating. The depolymerization rate and terephthalic acid conversion rate were as shown in Table 7.

[Table 7]

| | (a) PET sample 3 | (c) Solvent | | (b) Alkali compound | | Ratio of PET mass to solvent mass (a)/(c) | Depolymerization | | Depolymerization rate (%) | Terephthalic acid conversion rate (%) |
|---|---|---|---|---|---|---|---|---|---|---|
| | Mass (mol of repeating unit) | Type | Mass (Volume) | Type | Mass (Molar amount) | | Initial temp. | Time | | |
| Ex. 10 | 250 g (1.30 mol) | Methanol | 155 g (196 mL) | NaOH | 114 g (2.85 mol) | 1.61 | 50°C | 30 min | 95.7 | 94.6 |
| Ex. 11 | 250 g (1.30 mol) | Methanol | 155 g (196 mL) | NaOH | 114 g (2.85 mol) | 1.61 | 21°C | 30 min | 96.7 | 91.5 |
| Ex. 12 | 250 g (1.30 mol) | Methanol | 125 g (158 mL) | NaOH | 114 g (2.85 mol) | 2 | 19°C | 8 min | 95.4 | 97.9 |
| Ex. 13 | 250 g (1.30 mol) | Ethanol | 125 g (158 mL) | NaOH | 114 g (2.85 mol) | 2 | 15°C | 8 min | 73.8 | 74.2 |

**Claims**

1. A method for depolymerizing a polyester, the method comprising:

forming a mixture comprising the following components (a), (b), and (c):

(a) a polyester;
(b) one or more alkali compounds selected from the group consisting of an alkali metal hydroxide, an alkali metal carbonate, an alkali metal bicarbonate, an alkaline earth metal hydroxide, an alkaline earth metal carbonate, and an alkaline earth metal bicarbonate; and
(c) a solvent comprising at least one of an alcohol-based solvent or a ketone-based solvent,

and depolymerizing the polyester in the mixture,
wherein a temperature of the mixture at a time when the mixture comprising an entire amount of the polyester, the alkali compound, and the solvent is formed is 75°C or less, and
a ratio of an amount of the polyester to an amount of the solvent is from 0.2 to 20 on a mass basis.

2. The method according to claim 1, wherein the alkali compound is soluble in an amount of 100 g or more per 100 g of water at 20 ± 5°C.

3. The method according to claim 1, wherein the alkali compound is one or more alkali metal compounds selected from the group consisting of an alkali metal hydroxide, an alkali metal carbonate, and an alkali metal bicarbonate.

4. The method according to claim 1, wherein the alkali compound is an alkali metal hydroxide, an alkaline earth metal hydroxide, or both.

5. The method according to claim 1, wherein the alkali compound is an alkali metal hydroxide, an alkaline earth metal hydroxide, or both, and a total molar amount of hydroxide ions in the alkali metal hydroxide and hydroxide ions in the alkaline earth metal hydroxide is in a range of 0.9 to 3.0 relative to a molar amount of the polyester based on repeating units before the mixture is formed.

6. The method according to claim 1, wherein a temperature of the mixture at a time when the mixture comprising an entire amount of the polyester, the alkali compound, and the solvent is formed is from 0°C to 75°C.

7. The method according to claim 1, wherein a temperature of the mixture at a time when the mixture comprising an entire amount of the polyester, the alkali compound, and the solvent is formed is from 35°C to 55°C.

8. The method according to claim 1, wherein a ratio of an amount of the polyester to an amount of the solvent is from 0.25 to 2.2 on a mass basis.

9. The method according to claim 1, wherein the polyester is polyethylene terephthalate.

10. The method according to claim 1, wherein the alkali compound is sodium hydroxide, potassium hydroxide, or both.

11. The method according to claim 1, wherein the alcohol-based solvent is a lower alcohol.

12. The method according to claim 1, wherein the mixture comprises particles comprising the polyester.

13. The method according to claim 12, wherein the particles comprising the polyester are a granular material having an average particle size of from 150 to 350 μm.

14. The method according to claim 12, wherein a crystallinity of the particles comprising the polyester is 50% or less.

15. The method according to claim 12, wherein the mixture is a powdery mixture comprising the particles comprising the polyester and the solvent integrated with the particles.

16. The method according to claim 1, wherein the polyester before the mixture is formed has an intrinsic viscosity of 0.8 dl/g or less.

17. The method according to claim 1, wherein the polyester is prepared from used bottle containers comprising polyester.

18. A method for producing a diol and a dicarboxylic acid, the method comprising producing a diol and a dicarboxylate from a polyester by the method according to any one of claims 1 to 17.

19. A method for recycling a polyester, comprising producing a polyester by polymerization of a polymerization raw material comprising at least one of the diol obtained by the method according to claim 18, or a dicarboxylic acid produced from the dicarboxylate obtained by the method according to claim 18.

## INTERNATIONAL SEARCH REPORT

| International application No. |
| --- |
| **PCT/JP2024/016555** |

**A. CLASSIFICATION OF SUBJECT MATTER**

*C08J 11/16*(2006.01)i; *C07C 27/02*(2006.01)i; *C07C 31/20*(2006.01)i; *C07C 63/26*(2006.01)i; *C08J 11/24*(2006.01)i
FI:   C08J11/16; C07C31/20 A; C07C63/26 A; C07C27/02 ZAB; C08J11/24 CFD

According to International Patent Classification (IPC) or to both national classification and IPC

**B. FIELDS SEARCHED**

Minimum documentation searched (classification system followed by classification symbols)

C08J11/16; C07C27/02; C07C31/20; C07C63/26; C08J11/24

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2022/150016 A1 (AGENCY FOR SCIENCE, TECHNOLOGY AND RESEARCH) 14 July 2022 (2022-07-14)<br>p. 6, lines 10-21, p. 6, lines 31-36, p. 7, lines 26-28, p. 15, lines 27-30, p. 17, lines 21-28, p. 21, line 9 to p. 22, line 5, table 2 | 1-19 |
| A | JP 2007-519798 A (FREGOSO-INFANTE, Arturo Guadalupe) 19 July 2007 (2007-07-19)<br>entire text, all drawings | 1-19 |
| A | WO 03/042288 A1 (HITACHI CHEMICAL CO., LTD.) 22 May 2003 (2003-05-22)<br>entire text, all drawings | 1-19 |
| A | JP 8-253619 A (MATSUSHITA ELECTRIC INDUSTRIAL CO., LTD.) 01 October 1996 (1996-10-01)<br>entire text, all drawings | 1-19 |

☐ Further documents are listed in the continuation of Box C.      ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **01 July 2024** | **16 July 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

| Patent document cited in search report | Publication date (day/month/year) | Patent family member(s) | Publication date (day/month/year) |
|---|---|---|---|
| WO 2022/150016 A1 | 14 July 2022 | JP 2024-516332 A<br>EP 4288485 A1 | |
| JP 2007-519798 A | 19 July 2007 | US 2007/0219339 A1<br>entire text, all drawings<br>WO 2005/082826 A1<br>EP 1710226 A1<br>CA 2595393 A1<br>IL 176986 A<br>AU 2004316446 A1<br>KR 10-2006-0127925 A | |
| WO 03/042288 A1 | 22 May 2003 | US 2005/0027023 A1<br>entire text, all drawings<br>DE 10297453 B4<br>CN 1585798 A | |
| JP 8-253619 A | 01 October 1996 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10087529 A

**Non-patent literature cited in the description**

- **OKU**. *Polymer Journal*, 1997, vol. 29 (9), 708-712